(19) Europäisches Patentamt

European Patent Office

Office européen des brevets

(11) **EP 0 927 147 B1**

(12) **EUROPEAN PATENT SPECIFICATION**

(45) Date of publication and mention
of the grant of the patent:
**29.10.2003 Bulletin 2003/44**

(21) Application number: **97938276.9**

(22) Date of filing: **14.08.1997**

(51) Int Cl.⁷: **C07C 2/66**

(86) International application number:
**PCT/US97/14256**

(87) International publication number:
**WO 98/009929 (12.03.1998 Gazette 1998/10)**

(54) **ALKYLATION OF ORGANIC AROMATIC COMPOUNDS USING BETA ZEOLITE CATALYST**

ALKYLIERUNG ORGANISCHER AROMATISCHER VERBINDUNG MIT EINEM BETA-ZEOLIT ALS KATALYSATOR

ALKYLATION DE COMPOSES AROMATIQUES ORGANIQUES AU MOYEN D'UN CATALYSEUR ZEOLITIQUE BETA

(84) Designated Contracting States:
**BE DE ES FR GB IT NL**

(30) Priority: **09.09.1996 US 711117**

(43) Date of publication of application:
**07.07.1999 Bulletin 1999/27**

(73) Proprietor: **Catalytic Distillation Technologies
Pasadena, Texas 77507 (US)**

(72) Inventor: **SMITH, Lawrence, A., Jr.
Pasadena, TX 77507 (US)**

(74) Representative:
**Ebner von Eschenbach, Jennifer et al
Ladas & Parry,
Dachauerstrasse 37
80335 München (DE)**

(56) References cited:
**US-A- 4 891 458        US-A- 5 321 181**

**Description**

BACKGROUND OF THE INVENTION

Field of the Invention

The present invention relates to a process for the alkylation of organic aromatic compounds. More particularly the invention relates to a process for the concurrent alkylation and distillation of reaction components (reactants and products) in a catalyst bed wherein the catalyst also serves as the distillation structure. More particularly the invention relates to a process wherein beta zeolite is used as the catalyst portion of the catalytic distillation structure.

Related Art

Ethyl benzene and cumene have traditionally been produced by the reaction of benzene and the respective olefin, 1.e., ethylene and propylene in the presence of an acidic catalyst. In some known processes the catalyst is highly corrosive and has a relatively short life, e.g., $AlCl_3$, $H_3PO_4$ on clay, $BF_3$ on alumina, and others require periodic regeneration, e.g., molecular sieves. The exothermicity of the reaction and the tendency to produce polysubstituted benzene require low benzene conversions per pass with large volume recycle in conventional processes.

Recently a new method of carrying out catalytic reactions has been developed, wherein the components of the reaction system are concurrently separable by distillation, using the catalyst structures as the distillation structures. Such systems are described variously in U.S. Patents 4,215,011; 4,232,177; 4,242,530; 4,250,052; 4,302,356; and 4,307,254 commonly assigned herewith. Briefly, a structure described there is a cloth belt with a plurality of pockets spaced along the belt, which is then wound in a helix about a spacing material such as stainless steel knitted mesh. These units are then disposed in the distillation column reactor. In addition, commonly assigned U.S. Patent No. 4,443,559 discloses a variety of catalyst structures for this use and is incorporated herein.

More recently the method has been applied to aromatic alkylation as in commonly owned U.S. Pat. No.'s 4,849,569; 5,019,669; 5,043,506; 5,055,627; 5,086,193; 5,176,883; 5,215,725; 5,243,115; and 5,321,181. These patents all specifically disclose the use of A, X Y, L, erionite, omega and mordenite molecular sieves as the catalyst in the catalytic distillation structure . Additionally, U.S. Pat. No. 4,950,834 discloses a distillation column reactor having two separate beds of molecular sieve catalyst comprising one of an omega type molecular sieve and the other of a "Y" type molecular sieve.

Innes in U.S. Pat. No. 4,891,458 reports the use of a beta type zeolite in the alkylation of organic aromatics in a fixed bed having a partial, preferably a totally, liquid phase which exhibited less coking and longer life than other zeolites.

In a conventional fixed bed operation where there in no distillation going on concurrently in the reaction zone, one can expect that some catalysts will perform much better, because they do not coke up as fast as other catalysts. This type of improvement was observed in U.S. Pat. No. 4,891,458 noted above. For example in Example 6 of that patent LZY-82 showed a decline in ethyl benzene production compared to zeolite beta, for an unspecified period on stream. The comparison hereinbelow shows that in the CD mode LZY-82 performs in a superior fashion, i.e., 99+ EB purity at 1400 hours, a period of more than 100 fold greater than suggested by the patent. What is unexpected is the almost level performance of the zeolite beta at three times the number of hours on stream compared to the LZY-82 and almost no impurities for this period.

SUMMARY OF THE INVENTION

Briefly, the present invention is a process for the alkylation of organic aromatic compounds by contacting the organic aromatic compound and a $C_2$ to $C_{20}$ olefin in a distillation column reactor containing a beta zeolite catalytic distillation structure in a distillation reaction zone thereby catalytically reacting said organic aromatic compound and said olefin to produce an alkylated organic aromatic product and concurrently in said fixed bed fractionating the resultant alkylated organic product from the unreacted materials. The catalytic distillation structure provides both the catalytic sites and the distillation sites. The alkylated organic aromatic product is withdrawn from the distillation column reactor at a point below the fixed bed and unreacted organic aromatic compound may be taken off as an overhead.

More specifically the beta zeolite molecular sieve catalyst packing is of such a nature as to allow vapor flow through the bed, yet provide a sufficient surface area for catalytic contact as described in the previously noted U.S. Pat. No.s 4,443,559, 4,215,011 and,4,302,356 which are incorporated herein in their entirety. The catalyst packing is preferably arranged in the upper portion of the distillation column reactor, more preferably occupying about one-third to one half of the column and extending substantially to the upper end thereof.

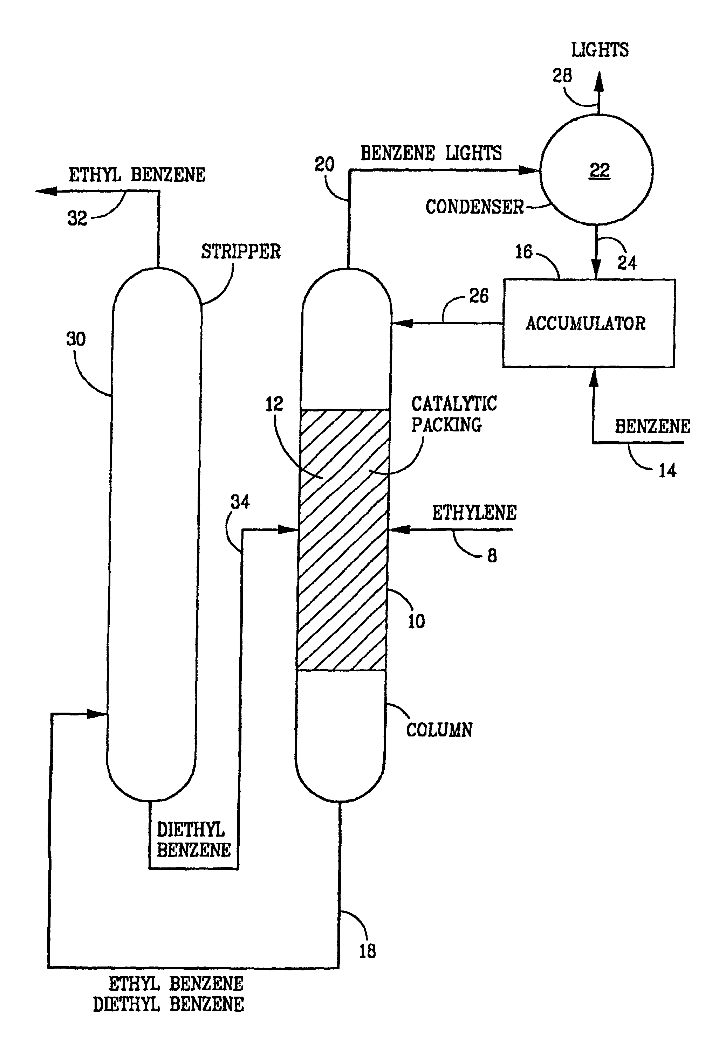

BRIEF DESCRIPTION OF THE DRAWING

The drawing is a schematic representation of a preferred embodiment of one species of the present invention for producing ethyl benzene.

DETAILED DESCRIPTION OF THE INVENTION

The exact location of the olefin feed will depend on the particular feeds and the desired product. In one embodiment the olefin feed to the reaction is preferably made below the catalyst bed thereby allowing mixing of the reactants before contact with the catalyst bed. In another embodiment the olefin feed to the reaction is preferably made into the catalyst bed thereby allowing immediate contact of this reactant with the organic aromatic compound in the catalyst to thereby react as much of the two as possible and reduce or eliminate the olefin leaving the reactor as overhead or bottoms, such as between the bottom of the fixed bed, and the upper one-fourth section thereof preferably in the middle one-half of the bed. For example, in the case of alkylation of benzene (B.P. 80°C) with propylene, the olefin feed may be located below the bed, whereas, for alkylation of benzene with decene (B.P.170°C) the decene is preferably fed into the upper half of the bed.

The organic aromatic compound feed may be added at any point in the reactor, however, preferably it is added to the fixed bed or to the reflux as makeup. Also, in order to achieve high selectivity toward monosubstitution (which is a preferred aspect of the present invention), there is a large excess of the organic aromatic compound to the olefin in the reactor in the range of 2 to 100 moles of organic aromatic compounds per mole of olefin, that is the net molar feed ratio of aromatic organic compound olefin may be close to 1:1, although the system is operated so as to maintain a substantial molar excess of organic aromatic compound to olefin in the reaction zone. The alkylated product is the highest boiling material and is separated in the lower portion of the column usually as bottoms. The organic aromatic compound can be the second highest boiling or third highest boiling component (excluding inerts) as noted above, however, by operating with a large excess of the organic aromatic compound, the major portion of the olefin is reacted: thereby reducing the separation and recovery problems. The success of catalytic distillation lies in an understanding of the principles associated with distillation. First, because the reaction is occurring concurrently with distillation, the initial reaction product is removed from the reaction zone as quickly as it is formed. The removal the alkylation product minimizes polysubstitution, decomposition of the alkylation product and/or oligomerization of the olefin. Second, because the organic aromatic compound is boiling, the temperature of the reaction is controlled by the boiling point of that component at the system pressure. The heat of the reaction simply creates more boil up, but no increase in temperature. Third, the reaction has an increased driving force because the reaction products have been removed and cannot contribute to a reverse reaction (Le Chatelier's Principle).

As a result, a great deal of control over the rate of reaction and distribution of products can be achieved by regulating the system pressure. Also, adjusting the through-put (residence time = 1/liquid hourly space velocity) gives further control of product distribution and degree of olefin conversion. The temperature in the reactor is determined by the boiling point of the liquid mixture present at any given pressure. The temperature in the lower portions of the column will reflect the constitution of the material in that part of the column, which will be higher than the overhead; that is, at constant pressure a change in the temperature of the system indicates a change in the composition in the column. To change the temperature the pressure is changed. Temperature control in the reaction zone is thus controlled by the pressure; by increasing the pressure, the temperature in the system is increased, and vice versa. It can also be appreciated that in catalytic distillation as in any distillation there is both a liquid phase (internal reflux) and a vapor phase. Thus, the reactants are partially in liquid phase which allows for a more dense concentration of molecules for reaction, whereas, the concurrent fractionation separates product and unreacted materials, providing the benefits of a liquid phase system (and a vapor phase system) while avoiding the detriment of having all of the components of the reaction system continually in contact with the catalyst which would limit the conversion to the equilibrium of the reaction system components.

The olefins may be $C_2$ to $C_{20}$ olefins, preferably $C_2$ to $C_{12}$ olefins, including normal and branched forms thereof. For example, suitable olefins are ethylene, propylene, butylene, isobutylene, 1-pentane, 1-hexane, 2-hexene, 2, 3-dimethyl-1-pentene, 1-octane, diisobutylene, 1-nonene and 1-decene, dodecene and the like.

The olefins may contain substituents which do not interfere with the alkylation. In one preferred embodiment the olefin is a $C_2$ to $C_4$ olefin.

In some reactions according to the present invention, the olefin will be a higher boiling material than the organic aromatic compound, e.g., $C_8$ to $C_{20}$ olefins. In such instances any unreacted olefin will appear in the bottoms alkylation product, although a side draw may be used to reduce such material in the product to an insignificant level. However, operating the reaction with far less than a stoichiometric amount of olefin in the reaction zone, as described, will normally keep the olefin. Level in the bottoms low or entirely eliminated.

In those instances wherein the olefin is lower boiling than the organic aromatic compound, e.g., $C_2$ to $C_7$, the

aromatic compound is in the large molar excess present in the reaction zone. In those instances the overhead may be condensed to recycle a major portion of the organic aromatic compound and the olefin and inerts removed for further separation or use. Similarly inerts such as the alkane of the particular olefin(s) which are often found in olefin streams will be a possible contaminant depending on its boiling point in either the bottoms or overhead.

The organic aromatic compounds are preferably those having a boiling point of 250°C or less under the pressure conditions of the distillation column reactor. The organic aromatic compounds include hydrocarbons of one or more rings and 6 to 20 carbon atoms which may contain substituents which do not interfere with the alkylation including halogen (Cl, Br, F and I), OH and alkyl, cycloalkyl, aralkyl and alkaryl radicals of 1 to 10 carbon atoms. Suitable organic aromatic compounds include benzene, xylene, toluene, phenol, cresol, ethyl benzene, diethyl benzene, naphthalene, indene, phenyl bromide, 1-bromo-2-chloro-benzene, 1-bromo-4-cyclohexyl benzene, 2-bromo-1,4-dihydroxy-benzene, 1 (bromo-methyl) naphthalene, 1,2-dihydronaphthalene and the like, a preferred group of compounds for use in the present process is benzene, xylene, toluene, phenol, and cresol.

The mole ratio of organic aromatic compound to olefin may be in the range of 2 to 100 : 1, preferably 2 to 50 : 1 and more desirably about 2 to 10 : 1. The greater the excess of organic aromatic compound the more the selectivity to the monosubstituted product is improved. Alkylation is forced to completion, since the simultaneous and concurrent fractionation and removal of the alkylation product from the distillation column reactor does not allow the products to contribute to the reverse reaction (Le Chatelier's Principle). However, very large molar excesses of organic aromatic compounds require a very high reflux ratio, and a low unit productivity. Hence, the correct ratio of organic aromatic compound to olefin must be determined for each combination of reactants as well as the acceptable olefin content in either the overhead or alkylation product (as described above), in a particular embodiment which is of current commercial importance ethylene or propylene is reacted with benzene according to the present invention to form ethyl benzene or cumene, respectively. In both of these reactions the olefin is the most volatile component and it is desirable to react it rather than have some carried off overhead. The presence of ethylene, propylene or other lower boiling olefin in the tower with benzene will result in a small but detectable temperature depression in the tower where such lower boiling olefins are present as entities and unreacted. As the ethylene, propylene or other lower boiling olefin in reacted with benzene, the depressing effect is diminished and furthermore, the reaction which is exothermic also diminishes the effect. The magnitude of the temperature depression immediately above the olefin feed is a measure of the concentration of ethylene or other lower boiling olefin in the system, that is, the larger the concentration of the lower boiling olefin, the greater the depression of the temperature where the benzene and olefin are initially together and yet unreacted. For any particular system the concentration of the olefin to provide a given temperature depression can be determined and plotted. Thus, by maintaining a specific temperature at the point of maximum temperature depression by adjusting the olefin feed, a given ratio of olefin to benzene can be maintained in a simple and expedient manner. More significantly, the maintenance of the depression at a given temperature can assure that substantially all of the olefin will be reacted prior to the end of the catalyst bed and overhead exit, if the corresponding, olefin concentration has been determined to produce that effect.

This same control system can be employed in regard to any combination of lower boiling olefin and higher boiling organic aromatic compound.

The length of the catalyst bed, particularly that portion wherein the reactants are in contact and the major portion of the reaction occurs, depends on the reactants, location of the olefin feed and the acceptable unreacted. Olefin in the streams leaving the tower. Some degree of development testing will be required for each set of reactants and parameters of stream purity following present disclosures.

The present alkylation reaction can be carried out at sub-through super atmospheric pressure, e.g., 20 to 4050 KPa (0.20 to 40 atmospheres). The temperature will vary depending on the reactants and product. Furthermore, the temperature along the column will be as in any distillation column, the highest temperature will be in the bottom and the temperature along the column will be the boiling point of the compositions at that point in the column under the particular conditions of pressure. Moreover, the exothermic heat of reaction does not change the temperature in the column, but merely causes more boil up. However, the temperatures within the column with the above considerations in mind will generally be in the range of 50°C to 500°C, preferably 70°C to 500°C, and more preferably in the range of about 80°C to 300°C at pressures of 51 to 2030 KPa (0.5 to 20 atmospheres).

In a preferred embodiment the reactor is operated with a liquid level in the reactor. The term "liquid level" is used herein to mean an increased density of the material in the reaction distillation zone over that of a pure distillation as distinguished to a continuous liquid phase. The phase system as present in the reaction distillation zone is physically a froth. This is the result of the vapor traveling up through the liquid retained in the zone.

Another way of viewing this is that in normal distillation there is a vapor with liquid (internal reflux) trickling down through the vapor and contracting the catalyst whereas in the present "flooded" system the vapor is traveling up through a liquid phase to create the froth or foam.

Hence in essence the benefits of the distillation are still obtained, i.e., separating the various components by the distillation whereas the increased liquid volume in contact with the catalyst improves the synthesis reaction.

Zeolite beta is a known synthetic crystalline aluminosilicate originally described in U.S. patent 3,308,069 which is hereby incorporated by reference for details of this zeolite, its properties and preparation. In particular, zeolite beta is identified by its characteristic X-ray diffraction pattern which is set out in Table 4 of the above referenced U.S.patent 3,308,069 This pattern, in terms of significant d values (Angstroms, radiation:K alpha doublet of copper, Geiger counter spectrometer), is reproduced in Table I below.

TABLE I
d Values of Reflection in Zeolite Beta

$11.4 \pm 0.2$

$7.4 \pm 0.2$

$6.7 \pm 0.2$

$4.25 \pm 0.1$

$3.97 \pm 0.1$

$3.0 \pm 0.1$

$2.3 \pm 0.1$

In U.S. patent 3,308,069 the zeolite beta is described in its as-synthesized forms as follows:

$$[XNa(1.0 \pm 0.1 - X)TEA]AlO_2 \cdot Y\ SiO_2 \cdot W\ H_2O$$

wherein X is less than 1, preferably less than 0.75, TEA represents tetraethylammonium ion, Y is greater than 5 and less than 100, and W is up to about 4, depending on the condition of dehydration and on the metal cation present, The patent also teaches that the sodium may be replaced by another metal ion suing ion exchange techniques.

Subsequent publications such as European Patent Applications Nos. 95,304; 159,846; 159,847 and 164,939 have broadened the definition of zeolite beta to include materials prepared using templating agents other than tetraethylammonium hydroxide and materials having Si/Al atomic ratios greater than 100. Also, the zeolites described in European Patent Application Nos. 55,046 ("Nu 2") and 64,328 and British Patent Application No. 2,024,790 ("Boralite B") have structures and X-ray diffraction patterns very similar to that of zeolite beta and are included within the scope of the term "zeolite beta" as used herein.

The forms of zeolite beta which are most useful in the present invention are crystalline aluminosilicates having the empirical formula:

$$(X/n)M \cdot (1.0 \pm 0.1 - X)Q \cdot AlO_2 \cdot Y\ SiO_2 \cdot W\ H_2O$$

wherein X is less than 1, preferably less than 0.75, Y is greater than 5 and less than 100, W is up to about 4, M is a metal ion, n is the valence of M, and Q is a hydrogen ion, an ammonium ion or an organic cation, or a mixture thereof. Y is preferably greater than 5 and less than about 50. Thus the silicon to aluminum atomic ratio in the above formula is greater than 5:1 and less than 100:1, and preferably greater than 5:1 and less than about 50:1.

It is also contemplated that other elements, such as gallium, boron and iron, can be substituted for aluminum in the above formula. Similarly, elements such as germanium and phosphorous can be variably substituted for silicon.

Suitable organic cations are those cations which are derived in aqueous solution from tetraethylammonium bromide or hydroxide, dibenzyl-1,4-diazabicyclo[2.2.2]octane chloride, dimethyldibenzyl ammonium chloride, 1,4-(1-azonium bicyclo[2.2.2]octane)butane dibromide or dihydroxide, and the like. These organic cations are known in the art and are described, for example, in European Patent Application Nos. 159,846 and 15,9847, and U.S. Pat. No. 4,508,837. The preferred organic cation is the tetraethylammonium ion.

M is typically a sodium ion from the original synthesis but may also be a metal ion added by ion exchange techniques. Suitable metal ions include those from Groups IA, IIA, IIIA of the Periodic Table or a transition metal. Examples of such ions include ions of lithium, potassium, calcium, magnesium, barium, lanthanum, cerium, nickel, palladium, and the like.

For high catalytic activity, the zeolite beta should be predominantly in its hydrogen ion form. Generally, the zeolite is converted to its hydrogen form by ammonium exchange followed by calcination. If the zeolite is synthesized with a high enough ratio of organonitrogen cation to sodium ion, calcination alone may be sufficient. It is preferred that, after calcination, a major portion of the cation sites are occupied by hydrogen ions and/or rare earth ions. It is especially preferred that at least 80% of the cation sites are occupied by hydrogen ions and/or rare earth ions.

In the pure powder form the zeolite beta forms too compact a bed and will not function adequately in a distillation, since there is a very large pressure drop through the bed and the free flow of internal reflux and rising vapor is impeded. Zeolite beta in the shape of conventional distillation structures, such as rings, saddles, and the like may be used in the present invention. The particulate zeolite beta may be employed by enclosing them in a porous container such as cloth, screen wire or polymeric mesh. The material used to make the container must be inert to the reactants and conditions in the reaction system. The cloth may be any material which meets this requirement such as cotton, fiber glass, polyester, nylon and the like. The screen wire may be aluminum, steel, stainless steel and the like. The polymer mesh may be nylon, teflon or the like. The mesh or threads per inch of the material used to make the container is such that the catalyst is retained therein and will not pass through the openings in thermal aerial. Particles of about 0.15 mm size or powders maybe used and particles up to about 6.4 mm (¼ inch) diameter may be employed in the containers.

The container employed to hold the catalyst particles may have any configuration, such as the pockets disclosed in the commonly assigned patents above or the container may be a single cylinder, sphere, doughnut, cube, tube or the like.

Each container containing a solid catalytic material comprises a catalyst component. Each catalyst component is intimately associated with a spacing component which is comprised of at least 70 volume % open space up to about 95 volume % open space. This component may be rigid or resilient or a combination thereof. The combination of catalyst component and spacing component form the catalytic distillation structure. The total volume of open space for the catalytic distillation structure should be at least 10 volume % and preferably at least 20 volume % up to about 65 volume %. Thus desirably the spacing component or material should comprise about 30 volume % of the catalytic distillation structure, preferably about 30 volume % to 70 volume %. Resilient materials are preferred. One suitable such material is open mesh knitted stainless wire, known generally as demister wire or an expanded aluminum. Other resilient components may be similar open mesh knitted polymeric filaments of nylon, teflon and the like. Other 5 materials such as highly open structures foamed material, e.g., reticulated polyurethane foam (rigid or resilient) may be formed in place or applied around the catalyst component. In the case of larger catalyst components such as from about 6.4 mm to 13 mm (¼ inch to ½) pellets, spheres, pills and the like each such larger component may be individually intimately associated with or surrounded by the spacing component as described above, it is not essential that the spacing component, entirely cover the catalyst component. It is only necessary that the spacing component intimately associated with the catalyst component will act to space the various catalyst components away from one another as described above. Thus, the spacing component provides in effect a matrix of substantially open space in which the catalyst components are randomly but substantially evenly distributed.

A preferred catalytic distillation structure for use herein comprises placing extruded zeolite beta particles into a plurality of pockets in a cloth belt, which is supported in the distillation column reactor by open mesh knitted stainless steel wire by twisting the two together in a helical form. This allows the requisite flows and prevents loss of catalysts the cloth may be an material which is inert in the reaction. Cotton or linen are useful: but fiber glass cloth or "Teflon" cloth are preferred.

In the following examples the catalyst packing consisted of bags in the form of a fiber glass cloth belt approximately six inches wide with narrow pockets approximately 19 mm (3/4 inch) wide sewn across the belt. The pockets are spaced about 6.4 mm (¼ inch) apart. These pockets are filled with the catalyst particles to form approximately cylindrical containers, and the open ends are then sewn closed to confine the particles. This belt is then twisted into a helical form to fit inside the column. Twisted in with the belt is also a strip of an open mesh knitted stainless steel wire, which serves to separate the mole sieve filled cloth pockets and provide a passage for vapor flow.

The wire mesh provides the support for the catalyst (belt) and provides some degree of vapor passage through the catalyst particles, which otherwise form a very compact bed which has a high pressure drop. Thus, the down flowing liquid is in intimate contact with the rising vapors in the column.

In commercial-scale operation, it is contemplated, catalyst packing would be made up of alternating layers of mole sieve filled cloth belts similar to the ones described above, and a spacing material which could be of any convenient, suitable substance, such as a corrugated wire screen or wire cloth or a knitted wire mesh. The layers would be arranged vertically or horizontally. For simplicity of fabrication and for better distribution of vapor flow passages, a vertical orientation is preferred. The height of a section of this packing should be of any convenient dimension, from a few inches to several feet. For ease of assembly and installation, the packing would be made into sections of the desired shape and size, each section fastened together with circumferential bands of tie wires depending on its size and shape. A complete assembly in a column would consist of several sections, arranged in layers, with possibly the orientation of the catalyst filled belts turned at right angles in successive layers to improve liquid and vapor flow distribution.

The drawing illustrates one species of the present invention, i.e., the production of ethylbenzene by alkylating benzene with ethylene and a preferred embodiment of that species. Referring to the drawing the distillation column reactor is divided into three sections. The catalyst packing (catalytic distillation structures) 12 are positioned in the middle section. The catalytic distillation structures contain either zeolite beta from PQ Corp. or a LZY-82 zeolite from UOP as a comparison deposited in the pockets of fiber glass belts and formed into a helix with stainless steel mesh as described.

The reactors 10 is a 76 mm (three inch) diameter pilot column 21m (70 feet) tall with 9m (30 feet) of the catalyst packing in the middle portion. The lower portion of the column is a conventional distillation column configuration (equivalent 25 trays). Benzene is conveniently added as makeup via 14 into reflux accumulator the benzene can also be added through a separate line (not shown). The ethylene is fed to the column via 8 at about the mid point of the catalyst packing 12 or below the catalyst bed (not show) for better mixing. The ethylene may also be fed at several points to reduce the concentration at any one location in the catalyst zone, thus reducing oligomerization as a side reaction. The reaction is exothermic and initiated by contacting the two reactants in the catalyst packing. Ethyl benzene and diethyl benzene are the principal reaction products. Both of these products are higher boiling than benzene and ethylene and are recovered via 18 as a bottoms product. The feed of ethylene is adjusted such that there is a molar excess of benzene in the reactor, such that the overhead 20 is primarily benzene, the ethylene having been almost totally reacted. In addition to benzene and some ethylene other lights go off overhead. The overhead is passed to condenser 22 which is operated to condense substantially all of the benzene which passes via 24 to accumulator 16 and hence, by reflux via 26 to column 10. The benzene used in the reaction and lost with the lights (which exit condenser 22 via 28) is made up by fresh benzene feed 14.

The bottoms contain a mixture of ethyl benzene and diethyl benzene which pass via 18 to splitter 30, which is a conventional distillation column operated to fractionate ethyl benzene and diethyl benzene. The ethyl benzene is recovered as overhead 32 and the diethyl benzene recovered as a bottoms product. In this preferred embodiment the diethyl benzene is returned via 34 to the lower portion of the catalyst packing 12 in column 10, although it could be recovered as such. However, in this preferred embodiment it is desired to maximize ethyl benzene production. There is an equilibrium between benzene and diethyl benzene in the catalyst as:

$$\text{Benzene} + \text{Diethyl Benzene} \leftrightarrow \text{Ethyl Benzene}$$

In the lower portion of the catalyst packing there is a large volume of benzene along with the reaction products and the recycled diethyl benzene, hence, the reversible reaction favors the production of ethyl benzene, which is being continuously removed from the catalytic zone.

Such conventional items as valves, reboilers, slip streams, etc. are not shown, but would be obvious expedients to those setting up such equipment.

EXAMPLES

A comparison of the feeds, conditions and results utilizing the two different zeolites in ethyl benzene columns is presented in TABLE II-IV below:

TABLE II

| FEEDS | | |
|---|---|---|
| EXAMPLE | 1-INVENTION | 2-COMPARISON |
| RUN | 1005 | 1016 |
| Run time, hours | 700-4200 | 0-1, 400 |
| BENZENE FEED, kgs/hr (lbs/hr) | 7.3 (≈ 16) | 7.3 (≈ 16) |
| Benzene purity, % | 99.8 - 99.95 | 99.97 - 99.99 |
| Lights, ppm | 500 - 1000 | 100 - 200 |
| Toluene, ppm | 100 - 110 | 35 - 70 |
| ETHYLENE FEED | | |
| Feed rate, kgs/hr (lbs/hr.) | 3 (6) | 3 (6) |
| Ethylene purity, % | 99.0 - 99.4 | 99.4 - 99.94 |
| Ethane, % | 0.2 - 0.3 | 0.05 - 0.6 |
| Propane, % | 0.6 | none |
| Propylene, % | none | none |

TABLE III

| CONDITIONS | | |
| --- | --- | --- |
| EXAMPLE | 1-INVENTION | 2-COMPARISON |
| RUN | 1005 | 1016 |
| Run time, hours | 700-4200 | 0-1,400 |
| Tower Diameter, mm (in.) | 76 (3) | 76 (3) |
| Cat. Height, mm (in.) | 760 (30) | 760 (30) |
| Cat. Weight, kgs (lbs.) | 10.2 (22.5) | 10.9 (24) |
| Catalyst type | Beta | LZY-82 |
| Cat. designation, mm | 6.3 (1/16") | 6.3 (1/16") |
| | PQ Corp. | UOP |
| Run time, hours | 700-4200 | 0-1, 400 |
| EXAMPLE | 1-INVENTION | 2-COMPARISON |
| Mid reflux, kgs (lbs/hr) | 59 (130) | 59 (130) |
| Column press. kPa (psig) | 1300 (175) | 1200 (160) |
| Column bed temp. °C (°F) | 202 (395) | 193 (380) |
| Selectivity: | | |
| EB/polyalkylated | 9-11/1 | 4-5/1 |

TABLE IV

| RESULTS | | |
| --- | --- | --- |
| EXAMPLE | 1-INVENTION | 2-COMPARISON |
| RUN | 1005 | 1016 |
| Run time, hours | 700-4200 | 0-1,400 |
| EB Product purity, % | 99.90-99.99 | 99.8-99.88 |
| Lights, wppm | 100-700 | 500 |
| Toluene, wppm | 100 | 400-600 |
| Xylene, wppm | 0 | 40-120 |
| Ethyl toluene, wppm | 0 | 20-50 |
| Cumene, wppm | 30 | 300-500 |
| n-propyl benzene | not measured | 20-100 |

When the impurities of the benzene feed are taken into account the only substantial by-product made by the zeolite beta is the cumene. Both catalyst exhibited the long life as a result of the catalytic distillation mode of operation.

**Claims**

1. A process for the alkylation of organic aromatic compounds comprising:

   (a) concurrently

      (i) contacting an excess of organic aromatic compound and a $C_2$ to $C_{20}$ olefin in a distillation column reactor containing a fixed bed of zeolite beta catalytic distillation structure in a distillation reaction zone wherein conditions of temperature and pressure cause vaporization in the distillation reaction zone to provide upward flowing vapors and downward flowing liquids thereby catalytically reacting said organic aromatic compound and said olefin to form an alkylation product having a high product purity and
      (ii) fractionating the resultant alkylation product and the unreacted organic aromatic compound and olefin in said fixed bed,

   (b) withdrawing said alkylation product from said distillation column reactor at a point below said fixed bed;

(c) withdrawing a small stream of unreacted aromatic compound as overhead; and
(d) condensing said aromatic compound overhead and returning substantially all of said aromatic compound overhead as reflux.

2. The process according to claim 1, wherein from 2 to 100 moles of organic aromatic compound per mole of olefin are present.

3. The process according to claim 2, wherein from 2 to 50 moles of organic aromatic compound per mole of olefin are present.

4. The process according to claim 3, wherein from 2 to 10 moles of organic aromatic compound per mole of olefin are present.

5. The process according to claim 1, wherein said organic aromatic compound has 6 to 20 carbon atoms.

6. The process according to claim 1, wherein said organic aromatic compound has a boiling point of 250°C or less under the pressure conditions in said distillation column reactor.

7. The process according to claim 1, wherein said pressure in said distillation column reactor is in the range of 25 to 4000 kPa (0.25 to 40 atmospheres).

8. The process according to claim 7, wherein the temperature is in the range of 80°C to 500°C.

9. The process according to claim 7, wherein said pressure is in the range of 51 to 2000 kPa (0.5 to 20 atmospheres).

10. The process according to claim 9, wherein the temperature is in the range of 80°C to 300°C.

11. The process according to claim 5, wherein said olefin is a $C_2$ to $C_7$ olefin.

12. The process according to claim 5, wherein said olefin is a $C_8$ to $_{20}$ olefin.

13. The process according to claim 5, wherein said organic aromatic compound is benzene, xylene, toluene, phenol or cresol.

14. The process according to claim 13, wherein said organic aromatic compound is benzene.

15. The process according to claim 13, wherein said organic aromatic compound is phenol.

16. The process according to claim 13, wherein said olefin is $C_2$ to $C_4$ olefin.

17. The process according to claim 14, wherein said olefin is ethylene.

18. The process according to claim 14, wherein said olefin is propylene.

19. The process according to claim 1, wherein said olefin is fed to said distillation column reactor at a point within said fixed bed.

20. The process according to claim 19, wherein said olefin has a lower boiling point than said organic aromatic compound, there being a temperature depression in said distillation column reactor at a point above the point at which said olefin is fed thereto by the mixture of said organic aromatic compound and olefin whereby a selected mole ratio of organic aromatic compound to olefin is maintained by adjusting the moles of olefin feed to maintain the temperature of said temperature depression at a predetermined point.

**Patentansprüche**

1. Verfahren für die Alkylierung organischer, aromatischer Verbindungen, umfassend:

(a) gleichzeitig

(i) Kontaktieren eines Überschusses an organischer, aromatischer Verbindung und eines $C_2$- bis $C_{20}$-Olefins in einem Destillationssäulenreaktor, der ein Festbett von beta-Zeolith als katalytische Destillationsstruktur in einer Destillationsreaktionszone enthält, worin die Bedingungen von Temperatur und Druck eine Verdampfung in der Destillationsreaktionszone bewirken, um nach oben strömende Dämpfe und abwärts strömende Flüssigkeiten zu schaffen, wodurch die organische, aromatische Verbindung und das Olefin unter Erzeugung eines Alkylierungsproduktes katalytisch umgesetzt werden, das eine hohe Produktreinheit hat;

(ii) Fraktionieren des resultierenden Alkylierungsproduktes und der nicht umgesetzten organischen, aromatischen Verbindung und des Olefins in dem Festbett;

(b) Abziehen des Alkylierungsproduktes aus dem Destillationssäulenreaktor an einer Stelle unterhalb des Festbettes:

(c) Abziehen eines kleinen Stroms von nicht umgesetzter aromatischer Verbindung als Überkopfprodukt; und

(d) Kondensieren der aromatischen Verbindung überkopf und Rückführen weitgehend gesamten aromatischen Verbindung, überkopf, als Rückfluss.

2. Verfahren nach Anspruch 1, bei welchem 2 bis 100 Mol organische, aromatische Verbindung pro Mol Olefin vorliegen.

3. Verfahren nach Anspruch 2, bei welchem 2 bis 50 Mol organische, aromatische Verbindung pro Mol Olefin vorliegen.

4. Verfahren nach Anspruch 3, bei welchem 2 bis 10 Mol organische, aromatische Verbindung pro Mol Olefin vorliegen.

5. Verfahren nach Anspruch 1, bei welchem die organische, aromatische Verbindung 6 bis 20 Kohlenstoffatome hat.

6. Verfahren nach Anspruch 1, bei welchem die organische, aromatische Verbindung unter den Druckbedingungen in dem Destillationssäulenreaktor einen Siedepunkt von 250°C oder weniger hat.

7. Verfahren nach Anspruch 1, bei welchem der Druck in dem Destillationssäulenreaktor im Bereich von 25 bis 4.000 kPa (0,25 bis 40 atm) liegt.

8. Verfahren nach Anspruch 7, bei welchem die Temperatur im Bereich von 80°C bis 500°C liegt.

9. Verfahren nach Anspruch 7, bei welchem der Druck im Bereich von 51 bis 2.000 kPa (0,5 bis 20 atm) liegt.

10. Verfahren nach Anspruch 9, bei welchem die Temperatur im Bereich von 80°C bis 300°C liegt.

11. Verfahren nach Anspruch 5, bei welchem das Olefin ein $C_2$- bis $C_7$-Olefin ist.

12. Verfahren nach Anspruch 5, bei welchem das Olefin ein $C_8$- bis $C_{20}$-Olefin ist.

13. Verfahren nach Anspruch 5, bei welchem die organische, aromatische Verbindung Benzol, Xylol, Toluol, Phenol oder Kresol ist.

14. Verfahren nach Anspruch 13, bei welchem die organische, aromatische Verbindung Benzol ist.

15. Verfahren nach Anspruch 13, bei welchem die organische, aromatische Verbindung Phenol ist.

16. Verfahren nach Anspruch 13, bei welchem das Olefin ein $C_2$- bis $C_4$-Olefin ist.

17. Verfahren nach Anspruch 14, bei welchem das Olefin Ethylen ist.

**18.** Verfahren nach Anspruch 14, bei welchem das Olefin Propylen ist.

**19.** Verfahren nach Anspruch 1, bei welchem das Olefin in dem Destillationssäulenreaktor an einer Stelle innerhalb des Festbettes eingespeist wird.

**20.** Verfahren nach Anspruch 19, bei welchem das Olefin einen niedrigeren Siedepunkt als die organische, aromatische Verbindung hat, wobei es eine Temperaturdepression in dem Destillationssäulenreaktor an einer Stelle oberhalb des Punktes gibt, bei der das Olefin in diesen mit Hilfe der Mischung der organischen, aromatischen Verbindung und des Olefins eingespeist wird, wodurch eine ausgewähltes Molverhältnis von organischer, aromatischer Verbindung zu Olefin aufrecht erhalten wird, in dem die Molzahl der Olefin-Beschickung so eingestellt wird, dass die Temperatur der Temperaturdepression an einer vorbestimmten Stelle aufrecht erhalten wird.

**Revendications**

**1.** Processus d'alkylation de composés aromatiques organiques comprenant :

(a) simultanément

(i) la mise en contact d'un excès de composé aromatique organique et une oléfine en $C_2$ à $C_{20}$ dans un réacteur à colonne de distillation contenant un lit fixe de structure de distillation catalytique de zéolite bêta dans une zone de réaction de distillation dans laquelle les conditions de température et de pression provoquent la vaporisation dans la zone de réaction de distillation pour fournir des vapeurs s'écoulant vers le haut et des liquides s'écoulant vers le bas, faisant ainsi réagir par catalyse ledit composé aromatique organique et ladite oléfine pour former un produit d'alkylation ayant une grande pureté de produit, et
(ii) le fractionnement du produit d'alkylation résultant et du composé aromatique organique qui n'a pas réagi et de l'oléfine dans ledit lit fixe,

(b) le retrait dudit produit d'alkylation dudit réacteur à colonne de distillation à un point en dessous dudit lit fixe ;
(c) le retrait d'un petit courant de composé aromatique n'ayant pas réagi comme un distillat de tête ; et
(d) la condensation dudit distillat de tête de composé aromatique et le renvoi sensible de tout ledit distillat de tête de composé aromatique comme reflux.

**2.** Processus selon la revendication 1, dans lequel de 2 à 100 moles de composé aromatique organique par mole d'oléfine sont présentes.

**3.** Processus selon la revendication 2, dans lequel de 2 à 50 moles de composé aromatique organique par mole d'oléfine sont présentes.

**4.** Processus selon la revendication 3, dans lequel de 2 à 10 moles de composé aromatique organique par mole d'oléfine sont présentes.

**5.** Processus selon la revendication 1, dans lequel ledit composé aromatique organique a 6 à 20 atomes de carbone.

**6.** Processus selon la revendication 1, dans lequel ledit composé organique a un point d'ébullition de 250°C ou moins dans les conditions de pression dans ledit réacteur à colonne de distillation.

**7.** Processus selon la revendication 1, dans lequel ladite pression dans ledit réacteur à colonne de distillation se trouve dans la plage de 25 à 4000 kPa (0,25 à 40 atmosphères).

**8.** Processus selon la revendication 7, dans lequel la température se trouve dans la plage de 80°C à 500°C.

**9.** Processus selon la revendication 7, dans lequel ladite pression se trouve dans la plage de 51 à 2000 kPa (0,5 à 20 atmosphères).

**10.** Processus selon la revendication 9, dans lequel la température se trouve dans la plage de 80°C à 300°C.

**11.** Processus selon la revendication 5, dans lequel ladite oléfine est une oléfine en $C_2$ à $C_7$.

**12.** Processus selon la revendication 5, dans lequel ladite oléfine est une oléfine en $C_8$ à $C_{20}$.

**13.** Processus selon la revendication 5, dans lequel ledit composé aromatique organique est le benzène, le xylène, le toluène, le phénol ou le crésol.

**14.** Processus selon la revendication 13, dans lequel ledit composé aromatique organique est le benzène.

**15.** Procédé selon la revendication 13, dans lequel ledit composé aromatique organique est le phénol.

**16.** Processus selon la revendication 13, dans lequel ladite oléfine est une oléfine en $C_2$ à $C_4$.

**17.** Processus selon la revendication 14, dans lequel ladite oléfine est l'éthylène.

**18.** Processus selon la revendication 14, dans lequel ladite oléfine est le propylène.

**19.** Processus selon la revendication 1, dans lequel ladite oléfine est alimentée au dit réacteur à colonne de distillation à un point à l'intérieur dudit lit fixe.

**20.** Processus selon la revendication 19, dans lequel ladite oléfine a un point d'ébullition inférieur à celui dudit composé aromatique organique, y ayant une baisse de température dans ledit réacteur à colonne de distillation à un point au-dessus du point auquel ladite oléfine est alimentée à ceci par le mélange dudit composé aromatique organique et de l'oléfine, moyennant quoi un rapport molaire choisi d'un composé aromatique organique/oléfine est maintenu en ajustant les moles d'oléfines alimentées pour maintenir la température de ladite baisse de température à un point prédéterminé.